# EUROPEAN PATENT APPLICATION

(11) **EP 2 018 857 A1**
(43) Date of publication of application: **28.01.2009**
(21) Application number: 07075640.8
(22) Date of filing: 23.07.2007
(51) Int. Cl.: A61K 31/351, A61K 31/665, A61K 38/12, A61L 15/44, A61L 24/00, A61L 27/54, A61L 31/16

(54) **Medical device and use of a pharmaceutical composition**

(71) Applicant: Biomet Deutschland GmbH, 14167 Berlin (DE)
(72) Inventor: Düwelhenke, Nicole, 14165 Berlin (DE)
(74) Representative: Gross, Felix

(57) **Abstract**

The invention relates to a medical device comprising on at least a part of its surface a pharmaceutical composition. According to the invention, said pharmaceutical composition comprises at least two polypeptide antibiotics or a combination of at least one polypeptide antibiotic and at least one further antibiotic, wherein said further antibiotic is a phosphonic antibiotic or mupirocin. The invention relates further to the use of a pharmaceutical composition as an additive to a medical device.

## Description

The invention relates to a medical device according to the preamble of claim 1 and to the use of a pharmaceutical composition according to the preamble of claim 11.

Due to demographic development, the incidence for primary joint replacements increases. The rate of infection for primary knee arthroplasty is about 5 % and for primary hip arthroplasty about 0.5 to 2 %. After revision operations, the infection rate is about 15 % in case of knee operations and about 2 to 5 % in case of hip operations. When looking at high-risk patients (patients having diabetes mellitus, rheumatoid arthritis, high overweight), the infection rates are already as high as 5 to 15 % in primary operations.

Infections of implanted prostheses are caused by a multitude of germs like Streptococci, Propionibacteria, *Pseudomonas aeruginosa,* Enterococci and in particular by *Staphylococcus aureus* and *Staphylococcus epidermidis.* In many cases, the infections are polymicrobial. Bacterial persistence is the rule in chronic osteomyelitis associated with a foreign body such as plate and screws or joint replacement. In such cases, antimicrobial therapy alone in often unsuccessful and the infection is cured only by implant removal and debridement of necrotic bone. In an animal model of orthopaedic implant infection it was demonstrated that as few as 50 organisms contaminating the operative site after a cemented hemiarthroplasty had been implanted resulted in infection, whereas 10000 organisms were necessary to produce infection in the absence of a foreign body.

There is evidence for a local host defence defect in the pathogenesis of foreign body infection. Polymorphonuclear leucocytes (PML) extracted from tissue fluid surrounding a foreign body are unable to kill catalase positive *S. aureus* despite optimal opsonisation. The same PMLs also exhibit a decreased production of superoxide and have lower content of enzymatic granules, an indication of impaired response to infection. The in vitro interaction of PML with Teflon leads to respiratory burst and exocytosis of enzymatic granules. The end result is a PML population composed of exhausted cells, with lower granule content, and less killing capacity. A similar study demonstrated that also the opsonisation of *S. aureus* in the presence of a foreign body is dramatically reduced at 20 hours. Since *S. aureus* is mainly killed by opsonisation and subsequent phagocytosis by PMLs, the host defence defects described above are particularly relevant to the development of infections associated with internal fixation or joint replacement.

To reduce the risk of infections, in prior art antibiotics (mainly cephalosporines like cefazolin) are administered systemically directly before operarations. Because cephalosporines do not diffuse well into bone tissue, locally achieved concentrations in bone tissue are much below the serum level. To achieve higher concentrations in the vicinity of the implant, bone cements containing gentamicin or tobramycin (both being aminoglycosides) are frequently used for cemented endoprotheses. A maximal protective effect could be shown for combined application of bone cement containing antibiotics and systemic antibiotics. But because of growing resistance to aminoglycosides, the prophylactic efficacy of these bone cements is decreasing. Staphylococci isolated over a 6-year period from patients with prosthetic joint infections were studied for aminoglycoside resistance. It was found that 41% of the isolates were resistant to gentamicin and 66% to tobramycin.

Since non-cemented prostheses are implanted in a still increasing number of cases, only prophylactic systemic application of antibiotics can be used for infection prophylaxis in these cases. Another possibility is an antibacterial coating of the implant, as is described in US 5,217,493. Here, a combination of minocycline (a tetracycline) and rifampin (a rifamycin), or a combination of novobiocin (an aminocoumarin) and rifampin is used to inhibit formation of biofilms of Staphylococci. Although the combination rifampin and minocycline was demonstrated to be very effective for the inhibition of biofilm formation on catheters and penile implants, the combination is suboptimal for orthopaedic or trauma implants for several reasons. Rifampin is the most effective antibiotic for the treatment of bone infections and especially prosthetic joint infections and should not be used for prophylaxis, because resistance against rifampin can occur very quickly. Tetracyclines, on the other hand, form insoluble complexes with calcium and precipitate in bone tissue. These complexes do not show antibacterial activity. Therefore, minocycline is most likely to be inactivated in bone tissue very fast. Additionally, minocycline is acting bacteriostatic and does not kill bacteria directly. Although novobiocin acts bactericidal against non-proliferating bacteria, it is considerd to be extremely toxic. In an animal model of an implant-associated infection in bone, the success rate for protection of the implant was only 50% for the rifampin/minocycline coating, while a coating with gentamicin had a 100% success rate in preventing implant-associated infections in another animal model, caused by inoculation of gentamicin-sensitive *S. aureus.*

It is an object of the invention to provide a medical device comprising a combination of pharmaceutical active substances being well suited in prophylactically treating infections.

This object is achieved by a medical device having the features of claim 1. Such a device comprises on at least a part of its surface a pharmaceutical composition, wherein the pharmaceutical composition comprises a combination of at least two polypeptide antibiotics or a combination of at least one polypeptide antibiotic and at least one further antibiotic, wherein said further antibiotic is a phosphonic antibiotic or mupirocin. Mupirocin is also known as pseudomonic acid A.

Mupirocin is effective against gram-positive bacteria, in particular against staphylococci and streptococci. Since it is not effective against most gram-negative bacteria, a combination with a polypeptide antibiotic like, e. g., polymyxin B or colistin, covering an effect against gram-negative bacteria is one possibility. Mupirocin inhibits bacterial protein biosynthesis and can thus be classified as bacteriostatic antibiotic. It also shows a bactericidal effect against staphylococci in a concentration-dependent manner after 24 hours of incubation. If it is used in connection with bone cement, it shows a bactericidal effect due to long exposure towards bacteria and high local concentrations of the antibiotic.

Since mupirocin has a half-life of only about 30 minutes in serum, it is not often used in systemic applications. Thus, only very few resistances against mupirocin exist.

In an embodiment of the invention, said phosphonic acid antibiotic is fosfomycin.

In another embodiment, the pharmaceutical composition further comprises at least one aminoglycoside antibiotic.

In a further embodiment of the invention, gentamicin, tobramycin, neomycin, amikacin and/or arbekacin are used as aminoglycoside. Aminoglycosides generally act bactericidal on non-proliferating bacteria and cover a part of the spectrum of gram-positive bacteria as well as a part of the spectrum of gram-negative bacteria to be eliminated.

As a possibility, at least one of the antibiotics chosen is known to hardly induce bacterial resistances against it.

As another possibility, at least one of the antibiotics chosen shows a bactericidal effect and does not only inhibit growth of bacteria. Otherwise, the bacteria being present on the device would re-grow after the antibiotics of the pharmaceutical composition are degraded or transported away from the device.

Since bacteria reduce their growth during transition from planktonic (floating) form into sessile form (i. e. when colonising the surface of an implant or another device), in one embodiment at least one of the antibiotics chosen should be bactericidal also against non-proliferating (resting) bacteria.

Antibiotics which only act bacteriostatic are not well suitable to prevent implant-associated infections, because they inhibit only the growth of bacteria. The bacteria were still needed to be eliminated by the host defence, which is impaired in the vicinity of bone implants, as described above. Other antibiotics like cephalosporins and vancomycin kill only proliferating bacteria and are inactive against resting (non-proliferating) bacteria. Therefore, particularly antibiotics which are bactericidal against non-proliferating bacteria can eliminate successful all bacteria and therefore have the highest potential in preventing implant-associated infections.

Unfortunately, only some antibiotics are bactericidal against non-proliferating bacteria: aminoglycosides, fluoroquinolones, rifampin, novobiocin, and some polypeptide antibiotics like polymyxins and daptomycin. Considering resistances and excluding reserve antibiotics like rifampin and daptomycin, a combination covering the whole bacterial spectrum of implant-associated infections causing bacteria and providing protection even against methicillin-resistant *Staphylococcus aureus* (MRSA) and methicillin-resistant *Staphylococcus epidermidis* (MRSE) comprises in an embodiment an aminoglycoside in combination with bacitracin and a polymyxin.

Aminoglycosides and polymyxins act bactericidal on non-proliferating bacteria, whereas bacitracin acts bactericidal only on proliferating bacteria, but acts synergistically with aminoglycosides. Although a lot of bacteria are resistant to aminoglycosides, resistances against bacitracin and polymyxin are very rare and occur very slowly even in vitro. Because both bacitracin and polymyxin are very nephrotoxic, they are in general not used for systemic treatment of infections, but they are suited to be used for the local application in bone or other tissue, because the resulting serum level of these antibiotics will be very low.

This triple combination of a polymyxin, bacitracin and an aminoglycoside is effective against all tested coagulase-negative Staphylococci, *Pseudomonas aeruginosa,* Enterobacteriaceae and 95% to 98% of *Staphylococcus aureus,* inclusive MRSA. Considering known resistance of antibiotics and antibacterial activity, this triple combination prevents in an embodiment more than 99% of all possible implant-associated infections.

In an embodiment of the invention, the surface of the device is coated in part or totally with said pharmaceutical composition, wherein a layer of said pharmaceutical composition can be applied directly onto the base material of the device (e. g. a Ti6Al4V alloy, another metal or a ceramic in case of an implant as device). In an alternative embodiment of the invention, the pharmaceutical composition is applied onto another layer being present on the device. Within the scope of the present invention is also a coating made of a support material in which the pharmaceutical composition is present (e. g. in a dispersed form). Such support material can be, e. g., polylactide. The support material with the dispersed pharmaceutical composition is then applied as coating onto the medical device (either directly onto the surface of the latter or onto a layer being already present on that surface or on another layer).

The pharmaceutical composition is, in one embodiment of the invention, used in such an amount that it is effective against the bacteria to be eliminated, especially effective in killing bacteria being causative for tissue and/or bone infections. Particularly, an amount of about 0.01 mg of the pharmaceutical composition per cm² of the device to about 10 mg per cm² is chosen. The concentrations can be also in arrange of about 0.1 mg per cm² to about 5 mg per cm² or about 0.5 mg per cm² to about 2 mg per cm².

In an embodiment of the invention, the polypeptide antibiotics are bacitracin and a polymyxin, wherein particularly polymyxin B is chosen. Bacitracin is effective against gram-positive bacteria by inhibition of cell wall synthesis; resistances against bacitracin occur hardly. However, a bactericidal effect of bacitracin acts only on proliferating bacteria.

Inhibition of cell wall synthesis results in a better uptake of aminoglycosides so that a combination of bacitracin and at least one aminoglycoside results in a synergistic effect.

Polymyxins are bactericidal against non-proliferating gram-negative bacteria. Development of resistances against polymyxin B occurs also only very rarely.

In an embodiment of the invention, the pharmaceutical composition further comprises a biofilm formation inhibitor. Every substance reducing or inhibiting at least partially the attachment of germs (especially bacteria) on a surface or the ability of germs to accumulate on a surface to form a biofilm on that surface is considered as biofilm formation inhibitor.

In an embodiment of the invention, salicylic acid or a pharmaceutical active derivative thereof is used as biofilm formation inhibitor. Particularly, a combination of salicylic acid and an aminoglycoside can be used since salicylic acid enhances the microbial activity of aminoglycosides against bacteria, especially against *E. coli* and *Klebsiella pneumoniae*: Salicylates enter a cell in a protonated form, thereby increasing the membrane potential of the cell. This, in turn, simplifies the uptake of aminoglycosides into the interior of the cell.

But even salicylic acid itself shows an effect on bacteria. E. g., growth of encapsulated *Klebsiella pneumoniae* in the presence of salicylate results in reduced synthesis of capsular polysaccharides. The loss of capsular material exposes the cell surface of *K. pneumoniae* to the host defence mechanisms, thus shortening the time required for infection clearance. Salicylic acid reduces the ability of bacteria to adhere onto surfaces and to form biofilms. Though salicylic acid does not provide 100 % protection against biofilm formation, it supports the effect of antibiotics.

Acetylsalicylic acid and/or its predominant metabolite, salicylic acid, exhibit definable impacts both *in vitro* and *in vivo* on microbial virulence phenotypes. Bacterial virulence factors help mediate infection by bacteria in a host organism. The following effects have been noted: reduction of adhesion to relevant biomatrices, reduction of capsule production, mitigation of biofilm formation, and diminution of vegetation growth, intravegetation bacterial proliferation, and hematogenous dissemination in experimental infective endocarditis. Salicylic acid also upregulates the translation of specific gene loci, including multiple antibiotic-resistance loci. Further, it induces cytoplasmic proteins; and increases quinolone resistance.

The synthesis of some types of fimbriae in *E. coli* (colonization factor antigen, P fimbriae and type 1 fimbriae) are reduced following growth in the presence of salicylate. Because fimbriae play a critical role in the attachment of *E. coli* to epithelial surfaces, salicylate treatment might prevent infection caused by some strains of fimbriated *E. coli*. Salicylate also limits adherence of *E. coli* to silastic catheters.

Chemotaxis in bacteria is modulated through regulation of flagella rotation. This rotation, when counterclockwise, leads to swimming along a linear trajectory and, when clockwise, leads to tumbling. Salicylate is recognized as a chemorepellant by the *E. coli* tsr gene product. This recognition leads to prolonged tumbling of motile *E. coli* and ultimately causes cells to migrate away from salicylate. Swarming behaviour of *E. coli* is also inhibited by salicylate in a concentration-dependent manner. Production of the flagellum itself in *E. coli* is inhibited by growth in the presence of salicylate. This is mediated by inhibiting the production of flagellin, the protein monomer constituting the flagella. It has also been speculated that inhibition of flagella synthesis and motility in *E. coli* by salicylate is due to reduced synthesis in OmpF synthesis, which may be required for flagella assembly.

Biofilms consist of microorganisms and other matter encased in a polysaccharide matrix of microbial origin. Growth of *Pseudomonas aeruginosa* and *Staphylococcus epidermidis* in the presence of salicylate reduces the production of extracellular polysaccharide required for biofilm formation. The reduction in biofilm formation decreases the ability of these organisms to adhere to contact lenses and medical polymers. A component of biofilm production in S. epidermidis is extracellular slime which is composed of a complex mixture of polysaccharides, teichoic acids and proteins. Production of slime-associated proteins and teichoic acids is inhibited in S. epidermidis by salicylate.

In case of *S. aureus,* salicylic acid mitigates two distinct virulence phenotypes that are of key relevance for matrix binding (i.e., to fibrinogen and fibronectin) and α-hemolysin activity. These effects are specifically associated with salicylic acid-mediated reduction in the expression of the respective structural genes (i.e., fnbA, fnbB, and hla). In addition to the suppression of matrix protein binding and cytolytic profiles, enhanced exoenzyme and protein A production occurs in the presence of salicylic acid. These findings raise the likelihood that salicylic acid executed its antimicrobial effects through one or more global regulatory networks rather than a decrease in general gene transcription. Global regulon sarA and the global regulon agr are mitigated by salicylic acid, corresponding to the reduced expression in of the hla and fnbA genes *in vitro.* It should be noted that *S. aureus* virulence parameters were not completely suppressed by salicylic acid but were reduced, in a drug concentration-dependent manner, by a maximum of approximately 50%.

The medical device can be coated with the pharmaceutical composition, or the pharmaceutical composition can be incorporated into the material of the medical device. In an embodiment, the medical device is an implantable prosthesis, wherein joint prostheses and particularly knee, hip, shoulder, elbow prostheses as well as vertebral implants are particularly chosen. Furthermore, all implants for trauma surgery like screws, plate, etc. may be used as medical device.

In another embodiment of the invention, the medical device is bone cement which might especially be used for fixing an implant in bone tissue.

In an embodiment of the invention, pharmaceutical compositions used for bone cement can differ from those used for implants. Since a coating of an implant is in general only active for a period not longer than 2 days (often even shorter - e. g. only some hours), an antibiotic being part of a coating of an implant should harm bacteria in a maximal efficient manner regardless of the actual state of the bacteria. E. g., an antibiotic being effective only against proliferating or growing bacteria might be not that efficient since many bacteria do not start proliferating or growing with the short time-frame in which the antibiotic is active. Therefore, antibiotics being bactericidal on resting (non-proliferating) bacteria are particularly chosen when they are intended to be used as part of a coating of an implant.

On the other hand, antibiotics being dispersed within bone cement may diffuse over a period of some weeks or even months from the inner of the bone cement to its surface (where they in general meet bacteria to be acted upon for the first time). Therefore, in this case, also antibiotics may be used which act bactericidal only on proliferating bacteria, since during such a long period of effect, all bacteria to be eliminated can finally be targeted.

A combination of bacitracin and a polymyxin like, e. g., polymyxin B or colisitin with or without an aminoglycoside can be used as an additive for bone cements. Because resistance against aminoglycosides can occur after use of aminoglycoside-containing bone cement for prosthetic joint arthroplasty, bacitracin and polymyxin B are used in an embodiment of the invention since for both latter antibiotics emergence of resistance is infrequent in therapy and occurs even in vitro very slowly.

Still further, a combination of fosfomycin and a polymyxin like, e. g., polymyxin B or colistin (polymyxin E₂) can be used in case of the medical device being a bone cement. Fosfomycin acts bactericidal only on proliferating or growing bacteria, as does bacitracin. In particular, fosfomycin and bacitracin each are effective against MRSA. A combination of fosfomycin and a polymyxin like polymyxin B or colistin (polymyxin E₂) covers the whole spectrum of bacteria to be eliminated. Fosfomycin has the further property of being the smallest known antibiotic and therefore diffuses easily through and out of bone cement. Due to its structural similarity to hydroxyl apatite, it reversibly binds to hydroxyl apatite and is, therefore, not transported away from the bone as quick as other antibiotics are. Further, fosfomycin is absolutely nontoxic, a pivotal point for long-term application.

A combination of mupirocin (the properties of which are explained above) and a polymyxin like, e. g., polymyxin B or colistin is also suited in case the medical device being a bone cement.

To allow a better diffusion especially of bacitracin out of bone cement, a pharmaceutically acceptable carrier cam be used as diffusion enhancer for either of the antibiotics used.

It should be noted that a triple combination of two polypeptides and an aminoglycoside might be not the first choice with respect to bone cements considering the long period of effect in case of antibiotics in bone cement (see above), since three antibiotics might be simply too strong for the subject to be treated with a bone cement comprising said antibiotics. Thus, though it is generally possible to use a triple combination, in an embodiment of the invention only two antibiotics are chosen in case of the medical device being a bone cement. It should further be noted that gentamicin might induce formation of small colony variants so that, in case of bone cement, gentamicin is not chosen as aminoglycoside antibiotic in an embodiment of the invention.

In an embodiment of the invention, the bone cement comprises polymethyl methacrylate (PMMA) as base material. The pharmaceutical composition can be dispersed in the base material or can be coated onto it. It is only important that the pharmaceutical composition is provided in such a form on or in the bone cement that it can act on bacteria in the surroundings of the bone cement. Whether it is released from the bone cement by desorption, solution, diffusion or another method is not of primary interest. In any case, adjuvants promoting or retarding antibiotics release from the bone cement (or from the medical device) can be also used.

Further, the substrate can be soaked with the pharmaceutical composition to be used. In still another embodiment, the pharmaceutical composition can be polymerised with the base material. Thus, it is possible to coat the substrate with the pharmaceutical composition and/or to incorporate the pharmaceutical composition into the substrate.

The object of the invention is further achieved by use of a pharmaceutical composition having the features of claim 11 as an additive to a medical device.

With respect to further embodiments of the invention, reference is made the explanations given above which are analogously applicable for the claimed use, particularly regarding the medical device to be used and the antibiotics to be chosen.

The invention further relates to a method for treating a subject, wherein a medical device is used comprising on at least a part of its surface a pharmaceutical composition, wherein the pharmaceutical composition comprises a combination of at least two polypeptide antibiotics or a combination of at least one polypeptide antibiotic and at least one further antibiotic, wherein said further antibiotic is a phosphonic antibiotic or mupirocin.

Said treatment particularly relates to a prophylactic treatment against infections of soft tissue and/or bone tissue and/or bone. These infections might occur due to a surgical operation, particularly due to an operation related to implanting an implant into a human or non-human body. Thus, the treatment might be applied to a dead or alive human or non-human body.

With respect to further embodiments of this aspect of the invention, reference is made the explanations given above which are analogously applicable for said method, particularly regarding the medical device to be used and the antibiotics to be chosen.

## Claims

1. Medical device, comprising on at least a part of its surface a pharmaceutical composition, **characterised in that** the pharmaceutical composition comprises a combination of at least two polypeptide antibiotics or a combination of at least one polypeptide antibiotic and at least one further antibiotic, wherein said further antibiotic is a phosphonic antibiotic or mupirocin.

2. Medical device according to claim 1, **characterised in that** said phosphonic antibiotic is fosfomycin.

3. Medical device according to claim 1 or 2, **characterised in that** said pharmaceutical composition further comprises at least one aminoglycoside antibiotic.

4. Medical device according to claim 3, **characterised in that** said aminoglycoside antibiotic is chosen from the group comprising gentamicin, tobramycin, neomycin, amikacin and arbekacin.

5. Medical device according to any of the preceding claims, **characterised in that** said polypeptide antibiotic is chosen from the group comprising bacitracin and polymyxins, particularly bacitracin, polymyxin B and colistin.

6. Medical device according to any of the preceding claims, **characterised in that** said pharmaceutical composition further comprises a biofilm formation inhibitor.

7. Medical device according to claim 6, **characterised in that** said biofilm formation inhibitor is salicylic acid or a pharmaceutically acceptable derivate or salt thereof.

8. Medical device according to any of the preceding claims, **characterised in that** it is an implantable prosthesis, in particular a hip prosthesis, a shoulder prosthesis, an elbow prosthesis, a knee prosthesis or a vertebral implant or an implant for trauma surgery.

9. Medical device according to any of claims 1 to 7, **characterised in that** it is a bone cement for fixing an implant in bone tissue.

10. Medical device according to claim 9, **characterised in that** said pharmaceutical composition is an integral part of the bone cement base material.

11. Use of a pharmaceutical composition comprising a combination of at least two polypeptide antibiotics or a combination of at least one polypeptide antibiotic and at least one further antibiotic, wherein said further antibiotic is a phosphonic antibiotic or mupirocin, as an additive to a medical device.

12. Use according to claim 11, **characterised in that** said phosphonic antibiotic is fosfomycin.

13. Use according to claim 11 or 12, **characterised in that** said pharmaceutical composition is dispersed in said medical device or is coated onto at least a part of a surface of said medical device.

14. Use according to any of claims 11 to 13, **characterised in that** said pharmaceutical composition further comprises an aminoglycoside antibiotic.

15. Use according to claim 14, **characterised in that** said aminoglycoside antibiotic is chosen from the group comprising gentamicin, tobramycin, neomycin, amikacin and arbekacin.

16. Use according to any of claims 11 to 15, **characterised in that** said polypeptide antibiotics are bacitracin and a polymyxin.

17. Use according to any claims 11 to 16, **characterised in that** said pharmaceutical composition further comprises a biofilm formation inhibitor.

18. Use according to any claims 11 to 17, **characterised in that** said biofilm formation inhibitor is salicylic acid.
